Europäisches Patentamt

European Patent Office

Office européen des brevets

(11) Publication number: **0 274 860**
**A1**

## EUROPEAN PATENT APPLICATION

(21) Application number: 87310730.4

(51) Int. Cl.⁴: **A61M 16/00**

(22) Date of filing: 07.12.87

(30) Priority: 17.12.86 GB 8630108
27.01.87 GB 8701708

(43) Date of publication of application:
20.07.88 Bulletin 88/29

(84) Designated Contracting States:
BE CH DE ES FR GR IT LI NL SE

(71) Applicant: MEDIMATIC LIMITED
6 Taverners Square Silver Road
Norwich NR3 4SY(GB)

(72) Inventor: Dougan, James Cummings
16 Front Street
South Creake Norfolk(GB)
Inventor: Hilton, Cyril Rex
2 Ferry Corner
Surlingham Norfolk(GB)

(74) Representative: Stone, Patrick
28 Edenside Drive
Attleborough Norfolk NR17 2EL(GB)

(54) Mouth to mouth resuscitation apparatus.

(57) A mouth to mouth resuscitator which comprises a donor mouthpiece (10) and a recipient mouthpiece (12) connected by a communicating tube (14) which incorporates an airpermeable element (30) of a material which excludes passage of liquid but permits passage of air between the mouthpieces in both directions.

EP 0 274 860 A1

*Fig.3*

# Mouth to Mouth Resuscitation Apparatus

The invention relates to a mouth to mouth resuscitation apparatus.

In any instance where a person stops breathing for any reason, it is well known that prompt action must be taken to try to re-start breathing as quickly as possible. This applies, for example, to patients in a hospital, where trained medical staff will try to re-start the breathing, as well as to cases of accidents, where unskilled passers-by often have to try as best they can to start the victim's breathing as soon as possible.

There are several different known methods of resuscitation, but the most popular and commonly practised is the so-called "kiss of life", in which direct mouth to mouth resuscitation is effected. However, in some cases there is reluctance to assist on the part of the helper due to the risk of infection from the victim. For example, ambulance men, firemen and policemen, lifeguards and other persons often involved in such life saving operations have become increasingly concerned about this risk in recent years, since a number of diseases which may be spread by body liquids such as saliva and/or blood have become more prevalent.

One mouth to mouth resuscitation apparatus has been available since 1959 and can be used to reduce risk of cross-infection. This apparatus is known as "The Brook Airway". The Brook Airway comprises a donor mouthpiece into which the operator exhales and a recipient mouthpiece for placing over the patient's mouth such that a projection holds the mouth open and lies over the tongue of the patient to hold it in position to keep the air passages free. Between the donor mouthpiece and the recipient mouthpiece extends a tube which includes within it a one-way valve and a complicated by-pass system such that the patient's exhaled air does not pass back to the operator but is diverted via the one-way valve to a by-pass outlet. The use of such an airway means there is no physical contact between the operator and patient and there is no passage of air from the patient to the operator. However, the one-way valve and by-pass are mechanical arrangements and the resultant structure is expensive. This means that such airways are not readily available and so in emergencies they are not often to hand. Thus, although well equipped professionals such as nurses or ambulance men may sometimes have such apparatus available for their use, non-professional first aid helpers usually have no access to such apparatus. Additionally, the valve in the airway significantly restricts air flow from the donor to the patient, reducing the effectiveness of the resuscitation operation, and the reliability of the valve action may be suspect following a long period of non-use of the apparatus.

Other valve dependant resuscitation apparatus including a donor mouthpiece for use by an operator and a recipient mouthpiece for placing into cooperation with the patient's mouth, the two mouthpieces being coupled by an airway, are known from U.K. Patent Specification No. 533297 and U.S. Specification No. 4535765.

The invention is characterised by an air permeable element fitted in the airway and which prevents passage of liquid at least in a direction from the recipient to the donor but permits two-way passage of air between the mouthpieces.

In this way, even when air passes from the patient to the operator, substantially no liquid, carrying with it the risk of infection, can pass to the operator from the patient.

Preferably the material used for the air permeable element is one which also prevents passage of liquid from the operator to the patient, so that both operator and patient are protected from the spread of disease.

One flexible sheet material which has the property of being air permeable but inhibiting passage of liquid from one face to another is that sold under the Trade Mark PERMAIR F by Porvair Limited of Riverside Industrial Estate, Estuary Road, King's Lynn, Norfolk, United Kingdom. The PERMAIR F material used can be one having a closed surface and an open surface or one having two closed surfaces, in which case the material is wholly non-absorbent to liquids. The material is manufactured from high molecular weight polyurethane, which will in fact only allow passage of liquids at pressures much higher than the pressures arising in mouth to mouth resuscitation apparatus.

It will be appreciated that other materials having similar properties to PERMAIR F may be used to constitute the air permeable element of the invention, and that the use of a flexible sheet material for the purpose is not essential. Porous PTFE is one possibility. Sintered high density polyethylene such as that known by the trade name VYON is another.

Preferably, however, the apparatus includes a bag of flexible sheet material, with the mouth of the bag sealed to the interior surface of the airway and the closed end of the bag directed towards and/or into the recipient mouthpiece. Thus, when the patient exhales into the recipient mouthpiece, the bag of material has a tendency to collapse slightly, reducing passage of air from the patient to the operator. In use, the operator exhales into the donor mouthpiece to inflate the patient's lungs and may then slightly lift the recipient mouthpiece during exhalation by the patient. However, there is in any case no danger of infection to the operator since the bag can allow air to pass from patient to operator but prevents any liquid from passing through it to the operator.

A bag or envelope as above described provides a larger area for passage of air than an element such as a disc. However, an alternative material with higher air permeability could be employed in the form of a flat diaphragm or disc. Another possible modification would be a small sheet of air permeable material presenting a barrier to liquid which is mounted and sealed to the interior surface of the airway to extend from one side thereof adjacent one mouthpiece to the opposite side thereof adjacent the other mouthpiece.

In general, it is preferred that the apparatus is used in conjunction with a nose-clip to prevent passage of air through the nose of the patient whilst mouth to mouth resuscitation takes place. In this context, it is possible, if desired, to provide a bore in the wall of the airway between the air permeable element and the recipient mouthpiece. In this way, when the patient exhales into the recipient mouthpiece, at least some air will pass out of the bore. Preferably the bore is small enough to allow it to be closed by placing a finger over it. Thus, the operator can close the bore while blowing into the donor mouthpiece and open the bore as the patient exhales. Typically the bore may have a 6 mm diameter.

Preferably the donor mouthpiece, airway and recipient mouthpiece are all made of injection moulded plastics material. Thus, the airway and mouthpieces may be made of four separate components, with each of the mouthpiece components and the airway being separately injection moulded. However, it is possible for the airway to be integrally moulded with one or both of the mouthpieces. The plastics material used for the airway and/or for the donor mouthpiece may be rigid but preferably there is flexibility in the material used for the mouth cooperating component of the recipient mouthpiece, to aid the operator in manoeuvring the apparatus into position. For example, the airway and donor mouthpiece may be made of hard polypropylene, whilst with regard to the recipient mouthpiece, which preferably includes a shield for fitting around the mouth and a curved projection for holding down the patient's tongue, the component forming said shield and projection may be made of relatively soft polyurethane such as E.V.A.

In one possible construction, the airway is not rigid but is collapsible to allow the apparatus to be packed and stored in a more compact state. For example, the airway walls may be made of corrugated form which will expand lengthways when air is blown into the airway through the donor mouthpiece.

The apparatus may include a secondary filter, preferably a moisture-absorbent filter, arranged between the donor mouthpiece and the airway. This secondary filter may be made from a fabric similar to that used in a dust mask or a surgeon's mask. Alternatively, the filter may be a sintered plastics disc.

Thus, the invention provides a lightweight and inexpensive apparatus which is sufficiently inexpensive as to be disposable and which can be easily carried or stored in vehicles or buildings ready for use in the case of collapse or accident involving loss of breathing. Moreover, the apparatus is substantially as efficient as conventional direct mouth to mouth resuscitation and is not subject to deterioration with long periods of storage without use.

Four embodiments of mouth to mouth resuscitation apparatus in accordance with the invention are now described by way of example, with reference to the accompanying drawings in which:

Figure 1 is a side elevation of a first embodiment;

Figure 2 is an end elevation of the first embodiment;

Figures 3 and 4 are axial cross-sectional views of the apparatus, in planes at right angles, respectively on the lines III,III and IV,IV of Figure 2;

Figure 5 is an axial cross-sectional view through a second embodiment;

Figure 6 is a side elevation of a third embodiment; and

Figures 7 and 8 are side elevational and axial cross-sectional views of a fourth embodiment, respectively.

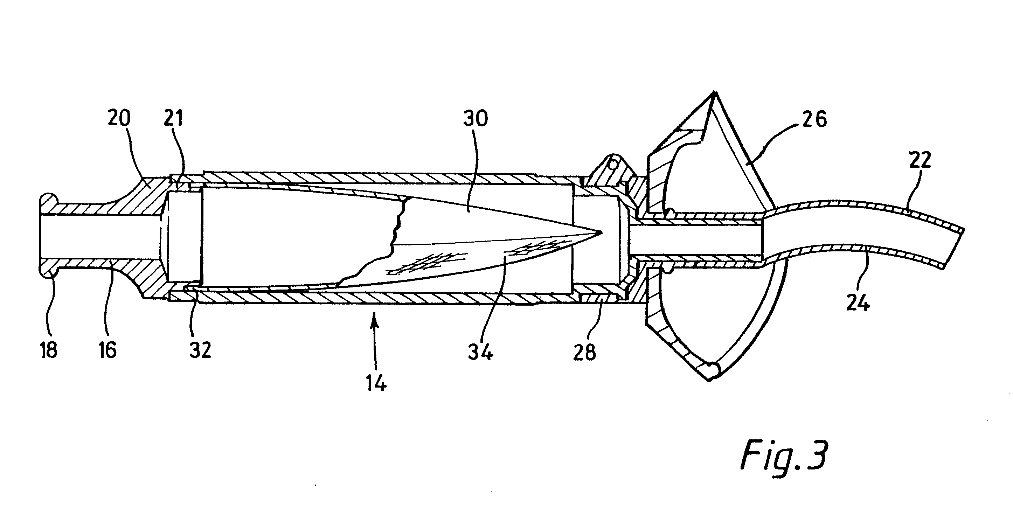

A first embodiment of mouth to mouth resuscitation apparatus comprises a donor mouthpiece 10 and a recipient mouthpiece 12 coupled by an airway 14. The donor mouthpiece 10 is in the general form of a cylindrical tube 16 shaped with a peripheral projection 18 to be inserted into the operator's mouth and with a wider base portion 20, which connects to a base fitting 21 (see Figure 3).

The recipient mouthpiece 12 has a relatively long curved projection 22 generally in the form of a somewhat flattened tube for inserting into the recipient's mouth. The curved surface 24 of the projection 22 serves in use to hold down the tongue of the patient, thereby to assist in keeping the patient's air passages open. The recipient mouthpiece 12 also has a relatively large somewhat cup-shaped shield or cover 26 to seal around the outside of the mouth of the patient. The shield 26 is preferably although not essentially produced as a separate component from the main portion of the mouthpiece which includes the projection 22, and is securely fitted to this main portion by locating it around the projection 22 against a base fitting 28.

The airway 14 comprises a hollow cylinder. Each of the components are separately injection moulded of suitable plastics material. Typically the plastics material used for the donor mouthpiece 10 will be relatively hard polypropylene, for example of hardness 74 degrees, and the recipient mouthpiece 12 will be of softer and more flexible polyurethane such as E.V.A., for example of hardness 55 degrees.

The donor mouthpiece 10 and the recipient mouthpiece 12 are assembled and substantially sealed to opposite ends of the airway 14, by means of base fittings 21 and 28.

Within the air passage 14 is mounted an air permeable element 30 which is in the general form of a bag, the mouth 32 of the bag 30 being sealed to the inside surface of the cylinder 14 with the closed end 34 of the bag directed towards the recipient mouthpiece 12. A preferred sheet material for the bag 30 is Permair F with a closed surface on the interior of the bag and an open surface on the outside. The closed surface presents a barrier to liquid, so that substantially no liquid originating with the recipient can pass to the donor operator. The bag is able to absorb liquid through its open outside surface, but if excessive liquid is produced by the patient the bag will saturate and, at the pressures prevailing in use in mouth to mouth resuscitation apparatus, become impervious even to air, which is a fail-safe condition to the operator. The material Permair F is sold by Porvair Limited of Riverside Industrial Estate, Estuary Road, King's Lynn, Norfolk, United Kingdom.

The thickness of the preferred material used is 0.5 millimetres, but a thickness of 0.7 or 1.2 millimetres would also be effective. Material with two closed surfaces may be employed or, in the case of material having a single closed surface, this may be on the outside of the bag. In all instances, the bag 30 is formed by heat sealing or plastics welding of the sheet material. As illustrated, the mouth 32 of the bag 30 is substantially sealed to the interior surface of the airway by trapping it between the cylinder 14 and the base fitting 21 of the donor mouthpiece 10. However, the illustrated method of assembly is exemplary only.

When the patient does exhale into the mouthpiece 12 the pressure on the air will tend to collapse the bag 30 slightly to reduce passage of air back to the operator, although it does not actually prevent the passage of air. The element 30 serves to prevent the passage of liquid so that no infection can pass between the patient and the operator.

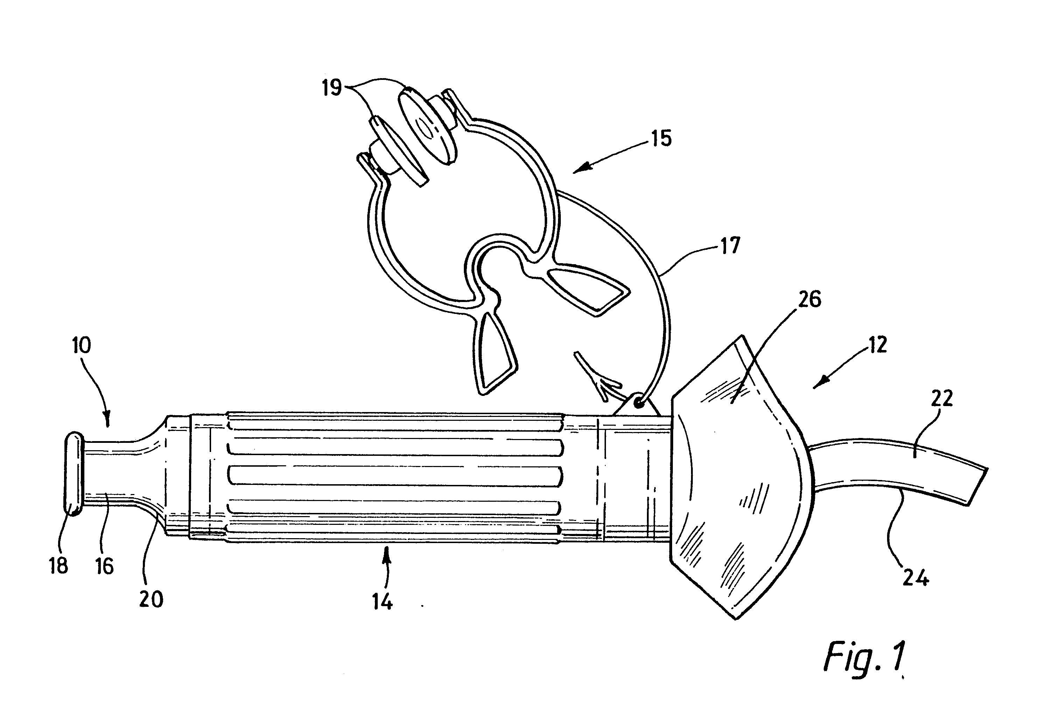

Figure 1 also serves to show a noseclip 15 loosely attached to the airway 14 adjacent the recipient mouthpiece 12 by a flexible plastics strand 17. The noseclip 15, conveniently made of plastics material such as acetal with soft nose pads 19 of a foamed plastics material such as T.P.R., serves to keep the nose of the patient closed during resuscitation, in accordance with preferred practice.

Thus, because the operator knows that there is significantly reduced danger of being infected from the patient, there will be no hesitation in using the mouth to mouth resuscitation apparatus and, therefore, no time will be lost in reviving the patient.

Because the product is lightweight and inexpensive it is readily portable and can be disposed of after use.

It is to be appreciated that where it is stated that the element 30 prevents passage of liquid therethrough, this refers to pressures which can be exerted by the donor or recipient in use of the apparatus. At very high pressures, well above the pressures which could be exerted by any donor or recipient, the material Permair F is able to allow a small degree of liquid passage.

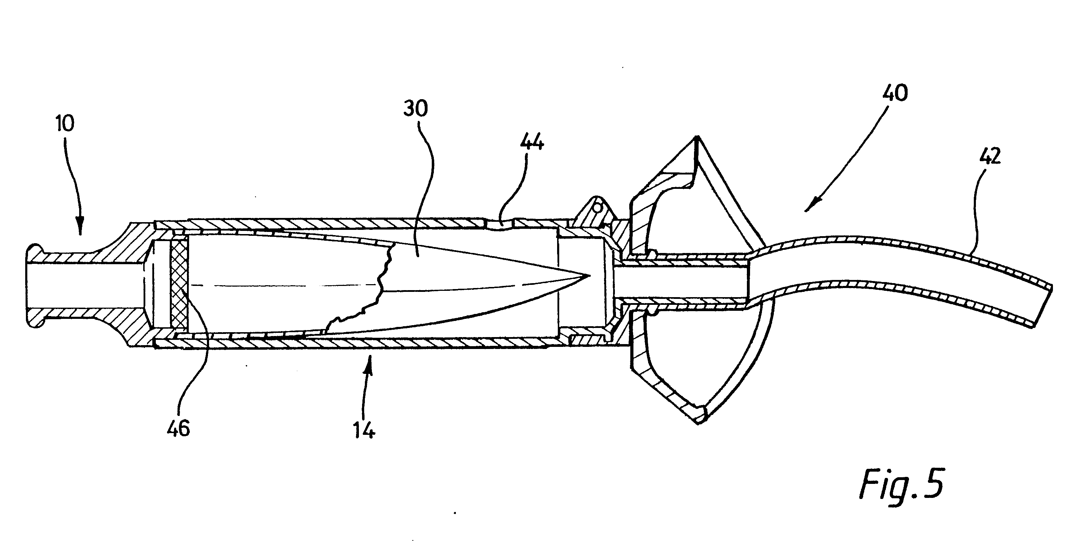

Figure 5 is a cross-section through a second embodiment taken in the same plane as the cross-section shown in Figure 3 of the first embodiment. The second embodiment shown in Figure 5 is very similar to the first apparatus in that the donor mouthpiece 10, the airway 14, and the element 30 are all identical to those of the first embodiment. However, the recipient mouthpiece 40 is slightly different. The curved projection 42 is longer than the curved projection of the first embodiment to ensure that not only is the tongue of the patient held down but also that the patient's airways are kept open. Additionally, the airway 14 includes a bore 44 which extends from inside the airway to outside the airway and which is positioned between the bag 30 and the recipient mouthpiece 40. The bore 44 has a diameter of six millimetres, which means that the operator can in fact readily close off the bore by placing a finger over it. When the donor blows into the mouthpiece 10 the bore is closed with the finger to allow free passage of the air through the bag 30 into the recipient mouthpiece 40. When the patient exhales the bore 44 is opened to allow most air to escape without returning to the donor. The air is prevented from being exhaled through the nose of the patient by use of the noseclip 15. The airway includes a secondary filter 46. This secondary filter is mounted within the airway 14 and as shown is positioned between the donor mouthpiece 10 and the air permeable bag 30. The secondary filter 46 may be a sintered plastics disc or an element of packed cotton wool or the like impregnated with silica gel, and most desirably acts as a moisture absorbent element.

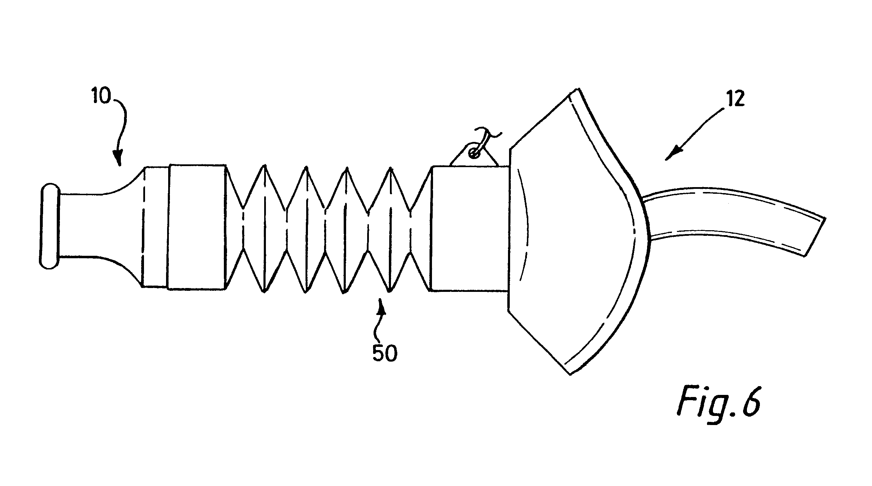

Figure 6 is a side elevation of a third embodiment which has the same donor mouthpiece 10, recipient mouthpiece 12 and air permeable bag 30 (not shown) as the first embodiment. However, the wall of the airway 50 is arranged to be collapsible. For example, the wall 50 is made of a plurality of flat rings of rigid polyurethane hinged together along lines of weakness to allow the walls to collapse in the manner of a concertina, thereby to allow the apparatus to be stored in a more compact state. When the apparatus is used, blowing into the donor mouthpiece 10 increases the pressure within the airway 50 and causes said airway to expand lengthways to its in-use state. Because the bag 30 is flexible, it can easily fold up within the collapsed apparatus. Alternatively the wall of the airway 50 could be of flexible corrugated material or it could be formed in two hinged together sections or even two or more telescopically interfitting sections.

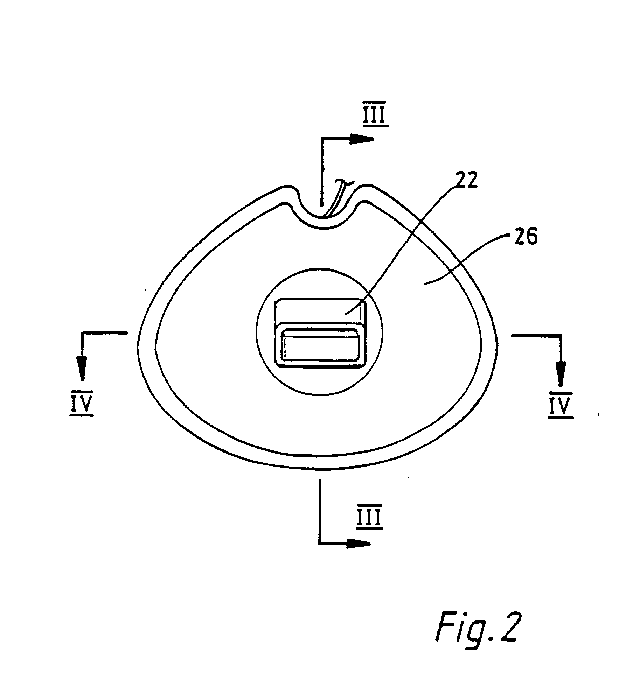

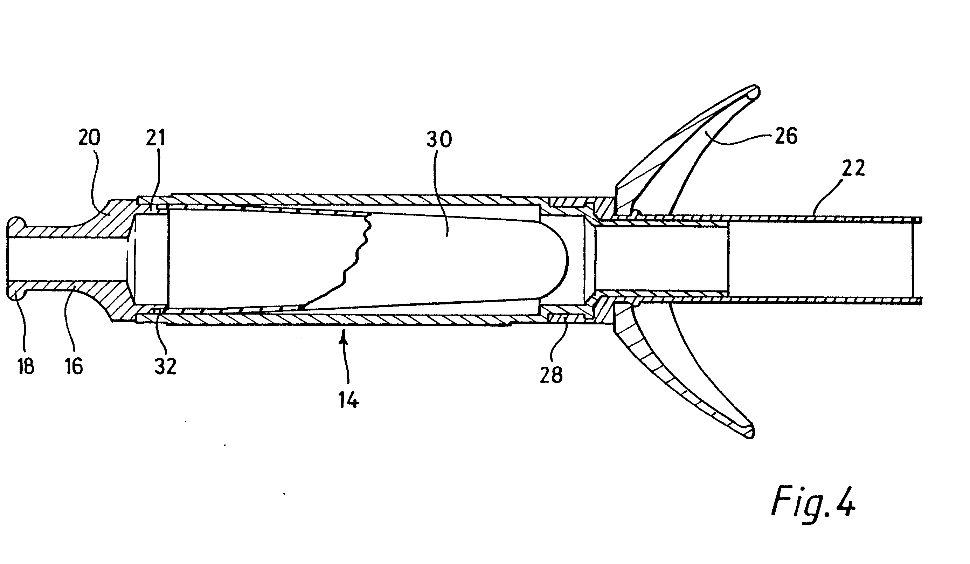

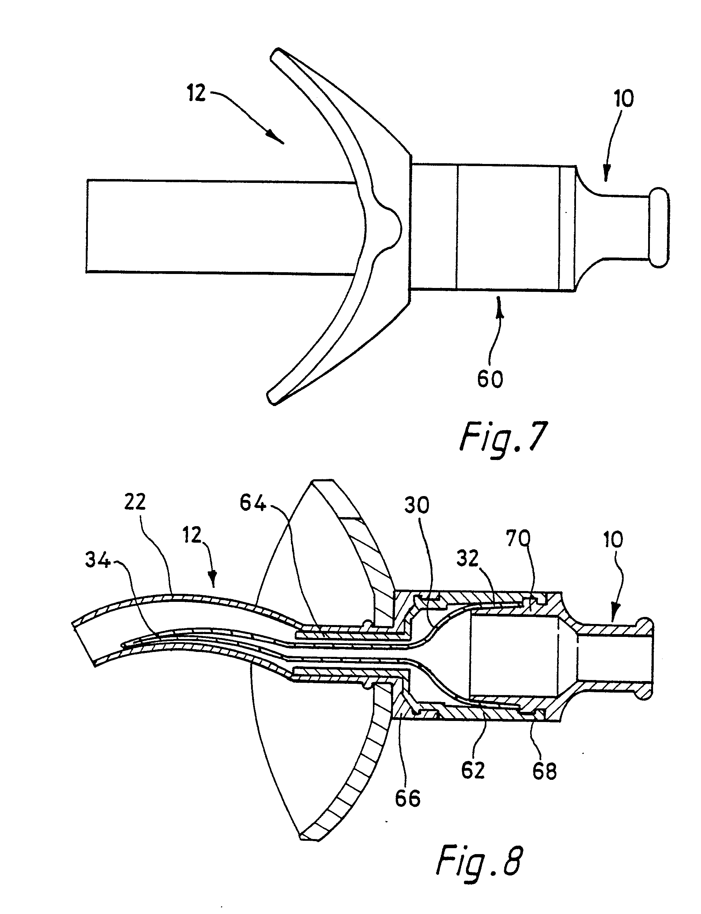

Figures 7 and 8 show a fourth embodiment having a donor mouthpiece 10 and recipient mouthpiece 12 generally of the same construction as that shown in Figures 1 to 4. However, the airway 60, whilst being rigid, is substantially shorter than the cylinder 14 of the first embodiment, so as to provide a more compact apparatus readily stored in a small space and easily carried around. The airway 60 comprises a relatively short cylindrical portion 62 of wider diameter and a relatively short cylinder portion 64 of reduced diameter which connects to the base portion 66 of the recipient mouthpiece 12. The end of the cylindrical portion 62 remote from the recipient mouthpiece 12 has a lipped formation 68 at which it is fitted to the base portion 70 of the donor mouthpiece 10. The air permeable element is a Permair F bag 30 substantially as described with reference to the first embodiment, with its mouth 32 substantially sealed to the interior wall of the airway 60 where the latter fits to the donor mouthpiece 10. However, the closed end 34 of the bag extends right through the airway 60 and into the curved, tubular projection 22 which forms part of the recipient mouthpiece 12.

Various further modifications of the above described and illustrated embodiments are possible within the scope of the invention as defined by the appended claims.

## Claims

1. Mouth to mouth resuscitation apparatus comprising a donor mouthpiece for use by an operator and a recipient mouthpiece for placing into cooperation with the patient's mouth, the two mouthpieces being coupled by an airway, characterised in that an air permeable element (30) is fitted within the airway for preventing passage of liquid at least in the direction from the recipient to the donor but permitting two-way passage of air between the mouthpieces.

2. Apparatus according to claim 1, characterised in that the air permeable element (30) also prevents passage of liquid in the direction from the donor to the recipient.

3. Apparatus according to claim 1 or claim 2, characterised in that the air permeable element (30) is made of a flexible sheet material, such as that sold under the Trade Mark PERMAIR F.

4. Apparatus according to claim 3, characterised in that the air permeable element (30) is in the form of a bag with the bag mouth being sealed to the inside of the airway and the closed end of the bag directed towards and/or into the recipient mouthpiece (12 or 40).

5. Apparatus according to any one of the preceding claims, characterised in that a wall of the airway (14) includes a bore (44) passing through from the inside of the airway to the outside at a position between the membrane and the recipient mouthpiece, the bore being sized such that it may readily be closed by a finger.

6. Apparatus according to any one of the preceding claims, characterised in that the donor mouthpiece (10), the airway (14) and the recipient mouthpiece (12 or 40) are made from injection moulded plastics materials.

7. Apparatus according to claim 6, characterised in that the donor mouthpiece (10) and airway (14) are made of a relatively hard plastics material and the recipient mouthpiece (12) includes a mouth cover (26) and a projection (22) for holding down the tongue made of a relatively soft plastics material.

8. Apparatus according to any one of the preceding claims, characterised in that the airway wall (50) is collapsible to allow the apparatus to be stored in a compact state.

9. Apparatus according to any one of the preceding claims, characterised in that a moisture-absorbent secondary filter (46) is mounted within the airway.

10. Apparatus according to any of the preceding claims, characterised by a nosepiece (15) loosely attached thereto adjacent the recipient mouthpiece(12).

Fig. 1

Fig.2

Fig.3

0 274 860

20  21  30

18  16  32  14  28  22  26

Fig.4

0 274 860

Fig.5

Fig.6

0 274 860

0 274 860

Fig.7

Fig.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US-A-3 057 347 (McGEE) <br> * Column 1, lines 7-10; column 2, lines 5-35; column 2, line 47 - column 3, line 3; figures 1,2,5 * | 1,2,6 | A 61 M 16/00 |
| Y | | 3-5 | |
| Y | US-A-4 510 931 (HENDERSON et al.) <br> * Column 3, line 64 - column 4, line 6; column 4, lines 20-23; column 4, lines 55-63 * | 3,4 | |
| Y | US-A-3 124 124 (CROSS) <br> * Column 2, lines 33-44; column 2, lines 57-70; column 3, line 65 - column 4, line 2; column 4, lines 40-45; figures 1,9 * | 4,5 | |
| A | | 6,7 | |
| A | GB-A-2 176 406 (VITALOGRAPH) <br> * Page 2, lines 15-22; page 2, lines 34-36; page 2, lines 43-45; page 2, lines 73-90; figures 1,6 * | 5,7 | |
| A | GB-A- 934 973 (McLEAN) <br> * Page 2, lines 1-6; page 2, lines 23-28; page 2, lines 44-49; figure 1 * | 4,8,9 | TECHNICAL FIELDS SEARCHED (Int. Cl.4) <br><br> A 61 M |
| X | FR-A-1 309 878 (ALBE) <br> * Whole document * | 1,2,5,7 ,10 | |

The present search report has been drawn up for all claims

| Place of search | Date of completion of the search | Examiner |
|---|---|---|
| THE HAGUE | 24-03-1988 | SCHOENLEBEN J.E.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)